# EUROPEAN PATENT SPECIFICATION

(11) **EP 3 485 877 B1**
(45) Date of publication and mention of the grant of the patent: **21.10.2026**
(21) Application number: 17828001.2
(22) Date of filing: 14.07.2017
(51) Int. Cl.: A61K 9/16, A61K 47/38, A61K 9/50, A61K 31/18, A61K 9/00

(54) **ORAL PHARMACEUTICAL PREPARATION HAVING IMPROVED CONTENT UNIFORMITY, CONTAINING TAMSULOSIN HYDROCHLORIDE-CONTAINING EXTENDED RELEASE PELLET**
ORALE PHARMAZEUTISCHE ZUBEREITUNG MIT VERBESSERTER GEHALTSUNIFORMITÄT MIT TAMSULOSINHYDROCHLORIDHALTIGEM RETARD-PELLET
COMPOSITION PHARMACEUTIQUE ORALE AVEC UNIFORMITÉ DU CONTENU AMÉLIORÉE, COMPRENANT UNE GRANULE À LIBÉRATION PROLONGÉE AVEC DU CHLORHYDRATE DE TAMSULOSINE

(30) Priority: 15.07.2016 KR 20160090266
(43) Date of publication of application: 22.05.2019
(73) Proprietor: Hanmi Pharm. Co., Ltd., Hwaseong-si, Gyeonggi-do 18536 (KR)
(72) Inventor: KIM, Hyung Seo, Hwaseong-si Gyeonggi-do 18587 (KR); CHO, Jung Hyun, Hwaseong-si Gyeonggi-do 18396 (KR); KIM, Jin Cheul, Seoul 07216 (KR); KIM, Yong Il, Suwon-si Gyeonggi-do 16325 (KR); PARK, Jae Hyun, Suwon-si Gyeonggi-do 16707 (KR); WOO, Jong Soo, Seoul 05555 (KR)
(74) Representative: Marks & Clerk LLP
(86) International application number: PCT/KR2017/007586
(87) International publication number: WO 2018/012931

(56) References cited:
- WO-A1-2005/077420
- WO-A2-2016/133333
- KR-A- 20050 074 767
- KR-A- 20050 074 767
- KR-A- 20050 082 038
- KR-A- 20070 044 911
- KR-A- 20110 113 940
- KR-A- 20160 100 570
- KR-B1- 100 745 515
- US-A1- 2005 106 253
- BANG KYO H: "KR20050074767A -SUSTAINED-RELEASE PELLET FOR TAMSULOSIN HYDROCHLORIDE AND THEIR METHOD OF PREPARATION", 19 July 2005 (2005-07-19), XP055841709, Retrieved from the Internet <URL:http://tfly.internal.epo.org/index.html?num=JP2005200399&type=PN&BNSDocId=JP%20%20%20%20%202005200399A%20%20I%20&BNSPage=10&FAM1=JP2005200399/TXPJPEA&FAM2=KR20050074767/TXPKA&FAM3=KR100570419B/TXPMTKEB> [retrieved on 20210916]

## Description

### [Technical Field]

The present invention relates to a method for preparing an oral pharmaceutical formulation comprising sustained-release pellets containing tamsulosin hydrochloride, and more particularly, to an oral pharmaceutical formulation comprising sustained-release pellets containing tamsulosin hydrochloride having improved content uniformity and deviation in dissolution rate and consistent efficacy.

### [Background Art]

Tamsulosin hydrochloride may selectively inhibit α-adrenoceptor to selectively act on the urogenital organ, thereby causing relaxation of smooth muscles surrounding the urinary bladder and prostate and improving urination rate, and is known having a good pharmaceutical efficacy in improving benign prostatic hypertrophy (BPH) symptoms with less side effects.

Tamsulosin hydrochloride has a relatively good bioavailability of 90% or greater, and has a half-life of about 9 to 13 hours in normal adults and a relatively long half-life of about 14 to 15 hours in benign prostatic hypertrophy patients. Accordingly, tamsulosin hydrochloride may maintain a sufficiently high concentration for 24 hours when prepared as a sustained-release formulation with prolonged release for only about 6 hours.

In general, tamsulosin hydrochloride is prescribed as a formulation containing 0.2 mg to 0.8 mg of tamsulosin hydrochloride in a single unit dosage form. However, due to a very low main ingredient proportion, it is crucial for this formulation to ensure content uniformity and low deviation in dissolution rate.

Patent document 1 discloses a capsule formulation containing sustained-release granules of tamsulosin hydrochloride, including tamsulosin hydrochloride, polyvinyl acetate, hydroxypropyl methylcellulose, and a granulating material. However, Patent document 1 discloses preparation of pellets without control of the amount of distilled water, so that the productivity of pellets and particle size uniformity may be reduced depending on the amount of the distilled water.

Patent document 2 discloses an oral sustained-release pellet composition of tamsulosin hydrochloride and a preparation method thereof. According to Patent document 2, the formulation may be prepared as granules by dissolving tamsulosin hydrochloride in distilled water and then adding the tamsulosin hydrochloride solution into a mixed solution of other ingredients. However, this preparation method of Patent document 2 may lead to a reduced content of tamsulosin hydrochloride, since the tamsulosin hydrochloride dissolved in distilled water may remain stuck to the walls of an equipment or container when added to the mixed solution of the other ingredients to form granules. In addition, a non-uniform distribution of the aqueous solution of tamsulosin hydrochloride dissolved in distilled water within the mixture may likely cause poor content uniformity of tamsulosin hydrochloride.

Therefore, there still is a need for the development of an oral pharmaceutical formulation of tamsulosin hydrochloride having a reduced deviation in dissolution rate and ensuring content uniformity. <PAGE 2A>

### [Prior art document]

### [Patent document]

1. KR 2005-0082038
2. KR 2005-0074767

### [Disclosure]

### [Technical Problem]

The present invention provides a method for preparing an oral pharmaceutical formulation including sustained-release pellets of tamsulosin hydrochloride in an appropriate proportion of pellets having different sizes, and thus having good content uniformity of tamsulosin hydrochloride and a reduced deviation in dissolution rate.

### [Technical Solution]

The scope of the present invention is defined in the claims.

Document US-A1-2005/106253 discloses coated pellets with a size between 0.3 and 0.85 mm. The pellet core is prepared from Tamsulosin, and Eudragit L, MCC and other compounds.

Document WO-A1-2005/077420 discloses enteric coated spheronized Tamsulosin granules comprising also polyvinylacetate for forming granules and creation of pores as well as HPMC, and microcrystalline cellulose as diluting agent. Said granules are prepared by passing the wet granule mass through a 0.8 mm mesh and spheronization, drying and coating.

According to an aspect of the present invention, there is provided a method for preparing an oral pharmaceutical formulation including sustained-release pellets containing tamsulosin hydrochloride and a pharmaceutically acceptable additive,
wherein the sustained-release pellets comprises 50 wt% to 100 wt% of particles having a particle size of 0.50 mm to 0.85 mm, and less than 15wt% of particles having a particle size less than 0.50 mm with respect to the total sustained-release pellets.

According to an aspect of the present invention, there is provided a method of preparing the above-described oral pharmaceutical formulation, the method including:
mixing and grinding a mixture of 10 parts to 300 parts by weight of polyvinyl acetate, 5 parts to 250 parts by weight of hydroxypropyl methylcellulose, and 1 part to 450 parts by weight of a diluting agent, with respect to 1 part by weight of tamsulosin hydrochloride, and 25wt% to 65wt% of distilled water based on a total weight of the forgoing ingredients, to form granules; and
spheronizing the formed granules with a spheronizer at a rotation speed of 600 rpm to 800 rpm for 15 to 35 minutes to obtain spheronized pellets.

### [Advantageous Effects]

As described above, according to the one or more embodiments, an oral pharmaceutical formulation including the tamsulosin hydrochloride-containing sustained-release pellets prepared by the method according to the present invention may exhibit stable efficacy due to a reduced deviation in dissolution rate and ensured content uniformity, compared to conventional tamsulosin hydrochloride formulations. Furthermore, due to high reproducibility of the tamsulosin hydrochloride pellets, quality of the formulation is ensured.

### [Description of Drawings]

FIG. 1 is a microscopy image of tamsulosin hydrochloride-containing sustained-release pellets prepared in Example 1; and
FIG. 2 is a microscopy image of tamsulosin hydrochloride-containing sustained-release pellets prepared in Comparative Example 1.
FIG. 3 is an image of the contents including tamsulosin and tadalafil taken out of the composite capsule formulation prepared in Example 15.

### [Mode for Invention]

Hereinafter, embodiments of the present disclosure will now be described in detail. Unless otherwise defined, all terms (including technical and scientific terms) used herein have the same meaning as commonly understood by one of ordinary skill in the art to which this invention belongs. Although exemplary methods or materials are listed herein, other similar or equivalent ones are also within the scope of the present invention. All numerical values represented herein are regarded including the meaning of "approximately" or "about", although not explicitly recited.

As a result of research into an oral pharmaceutical formulation including tamsulosin hydrochloride-containing sustained-release granules having a reduced deviation in dissolution of the active ingredient and content uniformity, the inventors of the present application have found that, when the sustained-release pellets include 50wt% to 100wt% of particles having a particle size of 0.50 mm to 0.85 mm and less than 15wt% of particles having a particle size less than 0.50 mm, the oral pharmaceutical formulation may have a reduced deviation in dissolution of the active ingredient tamsulosin hydrochloride and improved content uniformity.

Furthermore, the inventors of the present application have completed the present invention based on the finding that, when sustained-release pellets are prepared using polyvinyl acetate and hydroxypropyl methylcellulose each in a specific weight percent ratio and by adding distilled water in a certain weight percent with respect to a total weight of the tamsulosin hydrochloride and other additives, the pellets may have uniform particle size.

An aspect of the present invention provides a method for preparing an oral pharmaceutical formulation including sustained-release pellets containing tamsulosin hydrochloride and a pharmaceutically acceptable additive, wherein the sustained-release pellets includes 50 wt% to 100 wt% of particles having a particle size of 0.50 mm to 0.85 mm, and less than 15wt% of particles having a particle size less than 0.50 mm with respect to the total sustained-release pellets.

As the sustained-release pellets includes 50 wt% to 100 wt% of particles having a particle size of 0.50 mm to 0.85 mm, and less than 15wt% of particles having a particle size less than 0.50 mm, the oral pharmaceutical formulation may have a reduced deviation in dissolution rate of tamsulosin hydrochloride within the same batch, consistent content uniformity of the active ingredient, and consequently exhibit consistently stable efficacy of tamsulosin hydrochloride (see Test Examples 2 and 3).

In some embodiments, the sustained-release pellets may include 50 wt% to 100 wt%, 55 wt% to 95 wt%, or 60 wt% to 90 wt% of the particles having a particle size of 0.50 mm to 0.85 mm, and less than 15wt%, less than 10 wt%, less than 7 wt% of the particles having a particle size less than 0.50 mm. In some embodiments, the sustained-release pellets may include less than 50wt%, less than 45wt%, or less than 35wt% of particles having a particle size of 0.85 mm or greater.

Examples of the pharmaceutically acceptable additive may be a diluting agent, a disintegrating agent, and a binding agent that are commonly used in preparation of medicines, which may be used alone or a combination thereof. For example, the pharmaceutically acceptable additive may include a diluting agent, a disintegrating agent, and a binding agent all together.

To satisfy the above-described particle size ranges of the sustained-release pellets, any pharmaceutical means may be used. In some embodiments, a hydrophilic binding agent may be used to prepare the pellets, and sieving with a 0.85-mm (mesh size) sieve and a 0.5-mm sieve may be performed after preparation of the pellets.

In some embodiments, the sustained-release pellets containing tamsulosin hydrochloride may include 10 parts to 300 parts by weight of polyvinyl acetate, 5 parts to 250 parts by weight of hydroxypropyl methylcellulose, and 1 part to 450 parts by weight of a diluting agent, each with respect to 1 part by weight of tamsulosin hydrochloride, may maintain their particle size uniform by being prepared using 25 wt% to 65 wt% of distilled water with respect to a total weight of the forgoing ingredients.

In some embodiments, the sustained-release pellets containing tamsulosin hydrochloride, may include 10 parts to 300 parts by weight of polyvinyl acetate, 5 parts to 250 parts by weight of hydroxypropyl methylcellulose, 1 part to 450 parts by weight of a diluting agent, each with respect to 1 part by weight of tamsulosin hydrochloride, and may be prepared using 25 wt% to 65 wt% of distilled water with respect to a total weight of the forgoing ingredients, and
the sustained-release pellets include 50 wt% to a 100 wt% of particles having a particle size of 0.50 mm to 0.85 mm, and less than 15wt% of particles having a particle size less than 0.50 mm.

The oral pharmaceutical formulation prepared by the method of the present invention may be any solid formulation including the sustained-release pellets without damage, for example, in the form of granules, tablets, or capsules. For example, the oral pharmaceutical formulation may be in the form of a capsule including the sustained-release pellets.

In the oral pharmaceutical formulation according to any embodiments, the polyvinyl acetate (PVAc) may support a pellet-forming material and forming pores in the pellets after a period of time in an aqueous medium. For this reason, the amount of the polyvinyl acetate may play an important role in uniformly maintaining a particle size of the pellets.

Polyvinyl acetate may provide the oral pharmaceutical formulation with a consistent sustained release capability for an extended period of time in an aqueous medium regardless of pH level, and thus is an essential ingredient of the sustained-release pellets. The polyvinyl acetate may have an average molecular weight of 100,000 to 500,000.

In some embodiments, the polyvinyl acetate of the oral pharmaceutical formulation may be used alone or as a mixture with other materials, for example, in the form of powder or a diluted aqueous solution. For example, the polyvinyl acetate may be used as a mixture in powder form with any other water-soluble polymers such as polyvinylpyrrolidone. For example, Kollidon SR^{®} (available from BASF), a powder prepared by spray-drying a 8:2(w/w) mixture of polyvinyl acetate and polyvinylpyrrolidone, may be used.

In some embodiments, the polyvinyl acetate may be a diluted aqueous suspension of a mixture with other pellet-forming materials, for example, Kollicoat SR30D^{®} (available from BASF, solid content of about 30%) as a suspension of a mixture of polyvinyl acetate, polyvinylpyrrolidone, and sodium lauryl sulfate in water. Any material in any form including about 30% or more of polyvinyl acetate may be used as a source of the polyvinyl acetate. In some embodiments, the sustained-release pellets may include 10 parts to 300 parts by weight, and in some embodiments, 25 parts to 150 parts by weight of polyvinyl acetate with respect to 1 part by weight of tamsulosin hydrochloride. When the amount of the polyvinyl acetate exceeds any of these ranges, the sustained release of the active ingredient may be too delayed. When the amount of the polyvinyl acetate is below any of these ranges, a quick release, not sustained, of the drug may occur.

In an oral pharmaceutical formulation according to any of the embodiments, the hydroxypropyl methylcellulose (HPMC) may control the dissolution rate of the active ingredient in the sustained-release pellets. In particular, the hydroxypropyl methylcellulose may control the initial phase release of the active ingredient by forming pores as it is dissolved in an aqueous medium together with other ingredients, to thereby enable consistent sustained release of the active ingredient. The hydroxypropyl methylcellulose as a material having binding ability may have a great impact on the particle size uniformity of the pellets. The hydroxypropyl methylcellulose may have a viscosity of about 10,000 mPa.s (10,000 cPs) or greater, and in some embodiments, of 10,000 to 100,000 mPa.s (10,000 to 100,000 cPs), and in some other embodiments, 15,000 to 100,000 mPa.s (15,000 to 100,000 cPs). Examples of hydroxypropyl methylcellulose having a viscosity within these ranges are METOLOSE^{®} 60SH, 65SH, and 90SH (available from Shin-Etsu Chemical Co., Ltd., Japan). When the viscosity of the hydroxypropyl methylcellulose is below any of these ranges, it may be difficult to achieve sustained release of the active ingredient in the sustained-release pellets even with an increased amount thereof. For example, the amount of the hydroxypropyl methylcellulose in the sustained-release pellets may be 5 parts to 250 parts by weight, and in some embodiments, 5 to 100 parts by weight with respect to 1 part by weight of tamsulosin hydrochloride.

The diluting agent may be a material able to maintain the volume of the pellets constant. The diluting agent may be any diluting agents that are generally used in preparing pellets. The diluting agent may be selected from microcrystalline cellulose, lactose, inorganic carriers such as dibasic calcium phosphate, dibasic calcium phosphate dihydrate, and tribasic calcium phosphate, and any combinations thereof. For example, the amount of the diluting agent may be 1 part to 450 parts by weight, and in some embodiments, 1 part to 400 parts by weight, with respect to 1 part by weight of tamsulosin hydrochloride.

In some embodiments, the sustained-release pellets of the oral pharmaceutical formulation may include 25 parts to 150 parts by weight of polyvinyl acetate, 5 parts to 100 parts by weight of hydroxypropyl methylcellulose, and 1 part to 400 parts by weight of the diluting agent, with respect to 1 part by weight of tamsulosin hydrochloride.

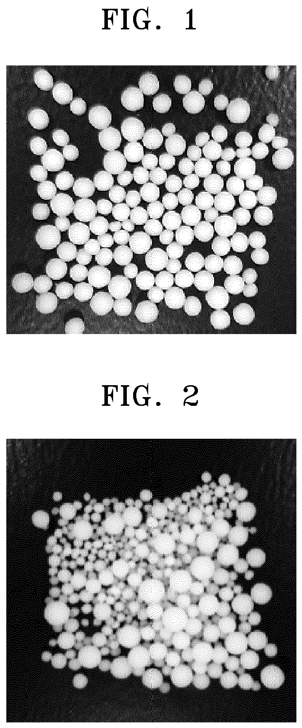

The sustained-release pellets of the oral pharmaceutical formulation may be prepared using 25 wt% to 65 wt% of distilled water based on a total weight of tamsulosin hydrochloride, polyvinyl acetate, hydroxypropyl methylcellulose, and the diluting agent. The amount of the distilled water used with respect to a total weight of the tamsulosin hydrochloride and the other additives may be 25 wt% to 65 wt%, 29 wt% to 61 wt%, 30 wt% to 60 wt%, or 35 wt% to 55 wt%. When the amount of distilled water used is less than 25wt% with respect to a total weight of the tamsulosin hydrochloride and the other additives, more small-size pellets may be formed with an increased deviation in particle size, leading to non-uniform particle sizes. On the other hand, when the amount of distilled water used is greater than 65 wt% with respect to a total weight of the tamsulosin hydrochloride and the other additives, the mixture may not be properly formed due to agglomeration of the mixture (see Test Example 1 and FIG. 2).

In some embodiments, by including the sustained-release pellets, the oral pharmaceutical formulation may exhibit sustained-release characteristics close to zero-order release, and may have a dissolution rate of the tamsulosin hydrochloride of 13% to 34% with a deviation of about 4.0 % or less with respect to the active ingredient during the dissolution test for about 2 hours in the pH 1.2 aqueous buffer solution, as evaluated using Dissolution method II (paddle method) in the U.S. Pharmacopoeia (USP) in 500 mL of a pH 1.2 aqueous buffer solution at about 37±0.5°C at about 100 rpm for about 2 hours. A formulation including a trace of an active ingredient tends to have a large deviation in dissolution rate between unit dosage forms. For such formulations, it is crucial to reduce a deviation in dissolution rate. In this regard, surprisingly the oral pharmaceutical formulation according to an embodiment may have a dissolution rate of 13% to 34%, which satisfies the reference level prescribed in the U.S. Pharmacopoeia (USP), with a range of about 21% between the lowest and highest levels, and a small deviation of about 4.0 % or less, for example, about 3.5 % or less.

In some embodiments, the sustained-release pellets may be additionally coated with a sustained-release coating material. The sustained-release coating material may be an enteric coating material or polymeric coating material that is commonly used in the art. For example, the enteric coating material may be selected from hydroxypropyl methylcellulose phthalate, hydroxypropyl methylcellulose acetate succinate, polyvinyl acetate phthalate, cellulose acetate phthalate, shellac, a methacrylic acid-methyl methacrylate copolymer, a methacrylic acid-ethyl acrylate copolymer, and any combinations thereof. For example, the polymeric coating material may be selected from hydroxypropyl methylcellulose, methylcellulose, ethylcellulose, polyvinyl acetate, and any combinations thereof.

In some embodiments, the amount of the sustained-release coating material may be 0.2 parts to 100 parts by weight, and in some other embodiments, 1 part to 50 parts by weight, with respect to 1 part by weight of tamsulosin hydrochloride.

In some embodiments, the sustained-release pellets after pelletization and an optional coating process may be formulated into any solid formulation without damage of the pellets using a common method known in the art. For example, the sustained-release pellets may be formulated as a capsule formulation.

In some other embodiments, the oral pharmaceutical formulation including the sustained-release pellets containing tamsulosin hydrochloride may be formulated as a composite formulation further including any additional active ingredient compatible with the tamsulosin hydrochloride. The additional active ingredient may be, for example, tadalafil, dutasteride, solifenacin, or finasteride.

In still other embodiments, the additional active ingredient may be enclosed as a separate form from the tamsulosin hydrochloride-containing sustained-release pellets in the oral pharmaceutical formulation. For example, the oral pharmaceutical formulation according to any of the embodiments may be formulated as tablets (double-layered tablets, drug-coated tablets), capsules (Polycap^{™}, drug-coated capsules), or composite granules.

For example, the oral pharmaceutical formulation according to any of the embodiments may be formulated as a Polycap^{™} formulation in which at least two active ingredients are enclosed as separate forms in a single capsule.

The oral pharmaceutical formulation according to any of the embodiments may be used for the treatment of any diseases which are known as an indication of tamsulosin hydrochloride, and for the treatment of any future diseases which will be identified as an indication of tamsulosin hydrochloride. As used herein, the expression "treatment" may include the meaning of "treatment", "improvement", "amelioration", and "management" of a disease. In some embodiments, the oral pharmaceutical formulation according to any of the embodiments may be used for the treatment of, for example, benign prostatic hypertrophy, urination disturbances concomitant with prostatic hypertrophy, and acute urinary retention.

Another aspect of the present invention, a method of preparing an oral pharmaceutical formulation according to any of the above-described embodiments includes:
mixing and grinding a mixture of 10 parts to 300 parts by weight of polyvinyl acetate, 5 parts to 250 parts by weight of hydroxypropyl methylcellulose, and 1 part to 450 parts by weight of a diluting agent, with respect to 1 part by weight of tamsulosin hydrochloride, and 25wt% to 65wt% of distilled water based on a total weight of the forgoing ingredients, to form granules; and
spheronizing the formed granules with a spheronizer at a rotation speed of 600 rpm to 800 rpm for 15 to 35 minutes to obtain spheronized pellets.

The above-detailed description of the oral pharmaceutical formulation according to any of the above-described embodiments may apply to description of the method of preparing the oral pharmaceutical formulation, subject-matter of the present invention.

The forming of the granules may be performed using any granulation method known in the art. In some embodiments, the forming of the granules may be performed by wet-grinding the ingredients and extrusion granulating the wet ground products. For example, the extrusion granulating may be performed using an extruder into which the wet ground products are put.

The spheronizing of the formed granules may be performed using a spheronizer at a rotation speed of 600 rpm to 800 rpm for 15 to 35 minutes. When the rotation speed and the time are below these ranges, the resulting sustained-release pellets may have a low sphericity and a particle size out of the desired ranges, leading to a low uniformity among the unit dosage forms. It may also be practically difficult to set the rotation speed above the range considering the condition of the spheronizer. When the spheronizing is performed for a time over the above range, the resulting sustained-release granules may have a reduced sphericity.

In some embodiments, the method of preparing an oral pharmaceutical formulation of any of the embodiments may further include filling a capsule with the spheronized sustained-release pellets to form a capsule formulation. For example, a pharmaceutically acceptable additive may be optionally added to the spheronized sustained-release pellets, if needed, which may then be filled into a hard capsule to form a capsule formulation. For example, the pharmaceutically acceptable additive may be a plasticizer, a lubricating agent, or any other adjuvants.

One or more embodiments of the present disclosure will now be described in detail with reference to the following examples. However, these examples are only for illustrative purposes and are not intended to limit the scope of the one or more embodiments of the present disclosure.

### [Examples]

### Examples 1 to 3 and Comparative Examples 1 to 3: Preparation of sustained-release pellets containing tamsulosin hydrochloride

Tamsulosin hydrochloride, polyvinyl acetate (PVAc) (Kollicoat^{®} SR30D, available from BASF), hydroxypropyl methylcellulose (HPMC) (METOLOSE^{®} 90SH), and a diluting agent were put into a high-speed mixer in a weight ratio as represented in Table 1, and an amount of distilled water represented in Table 1 was added thereto, mixed together for about 5 minutes to about 10 minutes, and then wet-ground. The resulting wet-ground product was put into an extruder equipped with a sieve having a mesh size of about 0.8 mm and extruded at a screw speed of about 35 rpm, followed by spheronizing with a spheronizer at a rotation speed of about 750 rpm for about 26 minutes to obtain core pellets.

With about 150.0 parts by weight of the tamsulosin hydrochloride-containing sustained-release pellets prepared above, a coating solution including about 5.5 parts by weight (1.7 parts by weight as a solid basis) of polyvinyl acetate (PVAc, Kollicoat^{®} SR30D), about 0.4 parts by weight of povidone, about 0.4 parts by weight of propylene glycol as a plasticizer, and about 16.0 parts by weight of distilled water was sprayed using a NQ-160 fluidized bed system (available from DALTON, Japan) from the bottom thereof. In addition, a coating solution including about 6.3 parts by weight (about 1.9 parts by weight as a solid ingredient) of a methacrylic acid-ethyl acrylate copolymer, about 0.5 parts by weight of triacetin, and about 40.0 parts by weight of distilled water was sprayed from the bottom of the same system, thereby preparing the sustained-release pellets of tamsulosin hydrochloride coated with the sustained-release coating agents. The spraying conditions were as follows: an inlet temperature of about 35-45°C, an outlet temperature of about 25-35°C, a spray rate of about 7 to 13 rpm, and an spraying air pressure of about 500-1000 m³/h.

**[Table 1] Composition of tamsulosin hydrochloride-containing sustained-release pellets (unit: mg)**

| Example | Sustained-release coating agents | | Main ingredient | Granulating material | Distilled water |
|---|---|---|---|---|---|
| | PVAc | HPMC | Tamsulosin hydrochloride | Microcrystalline cellulose | |
| Example 1 | | | | | 65 |
| Example 2 | | | | | 45 |
| Example 3 | | | | | 90 |
| Comparative Example 1 | 20.0 | 5.1 | 0.4 | 124.5 | 15 |
| Comparative Example 2 | | | | | 100 |
| Comparative Example 3 | | | | | 120 |

FIG. 1 is a microscopy image of tamsulosin hydrochloride-containing sustained-release pellets prepared in Example 1.

### Test Example 1: Evaluation of pellet particle size

The tamsulosin hydrochloride-containing sustained-release pellets of Examples 1 to 3 and Comparative Examples 1 to 3 were sieved through a sieve having a mesh size of about 0.85 mm and a sieve having a mesh size of about 0.5 mm to separately collect the particles remaining on the 0.85-mm sieve, the particles remaining on the 0.5-mm sieves, and the particles passed through the 0.5-mm sieve, as particles having a particle size of about 0.85 mm or greater, particles having a particle size of about 0.50 mm to about 0.85 mm, and particles having a particle size of less than about 0.50 mm, respectively, followed by calculating a percentage by weight of the particles having the different ranges of particle sizes. The results are shown in Table 2.

**[Table 2] Distribution of pellet particle sizes (unit: wt%)**

| Example | Particles of 0.85 mm or greater | Particles of 0.50 mm to 0.85 mm | Particles less than 0.50 mm |
|---|---|---|---|
| Example 1 | 24.4 | 74.0 | 1.6 |
| Example 2 | 23.1 | 73.1 | 3.8 |
| Example 3 | 20.1 | 79.0 | 0.9 |
| Comparative Example 1 | 33.1 | 48.7 | 18.2 |
| Comparative Example 2 | Pellets not properly formed due to agglomeration of the mixture | | |
| Comparative Example 3 | | | |

The tamsulosin hydrochloride-containing sustained-release pellets of Examples 1 to 3 were properly formed including about 50 wt% to about 100 wt% of particles having a particle size of about 0.50 mm to about 0.85 mm, and less than about 15wt% of particles having a particle size of less than about 0.50 mm. On the other hands, the pellets of Comparative Example 1 included a relatively high content (18.2%) of smaller pellets having a particle size less than about 0.50 mm. These test results indicate that, when using a small amount of distilled water, i.e., less than about 25wt% based on a total weight of tamsulosin hydrochloride and the other additives, as in Comparative Example 1, more small-size pallets may be formed with an increased deviation in particle size, leading to a non-uniform particle size. In this case, as can be expected from results of Test Examples 2 and 3, a high deviation in dissolution rate and low content uniformity occurred in Comparative Example 1. In Comparative Examples 2 and 3, agglomeration of the mixture occurred due to high content of the distilled water, and pellets were not properly formed.

### Examples 4 to 14 and Comparative Examples 4 to 8: Preparation of sustained-release pellets containing tamsulosin hydrochloride

The sustained-release pellets of Example 1 were sieved through a sieve having a mesh size of about 0.85 mm and a sieve having a mesh size of about 0.5 mm to separately collect the particles remaining on the 0.85-mm sieve, the particles remaining on the 0.5-mm sieves, and the particles passed through the 0.5-mm sieve. Next, the particles having the different particle sizes were mixed using a Turbula^{®} mixer (T2F, Switzerland, 75rpm, 15min) in a proportion as represented in Table 3 and then filled into capsules using a capsule charger, thereby preparing capsules formulations (Examples 4 to 14 and Comparative Examples 4 to 8).

**[Table3] Proportion of particles having different sizes in each capsule (unit: wt%)**

| Example | Particles of 0.85 mm or greater | Particles of 0.50 mm to 0.85 mm | Particles less than 0.50 mm |
|---|---|---|---|
| Example 4 | 45 | 55 | 0 |
| Example 5 | 35 | 55 | 10 |
| Example 6 | 35 | 65 | 0 |
| Example 7 | 25 | 65 | 10 |
| Example 8 | 25 | 75 | 0 |
| Example 9 | 15 | 75 | 10 |
| Example 10 | 15 | 85 | 0 |
| Example 11 | 5 | 85 | 10 |
| Example 12 | 5 | 95 | 0 |
| Example 13 | 0 | 100 | 0 |
| Example 14 | 20 | 67 | 13 |
| Comparative Example 4 | 0 | 80 | 20 |
| Comparative Example 5 | 55 | 45 | 0 |
| Comparative Example 6 | 10 | 75 | 15 |
| Comparative Example 7 | 20 | 65 | 15 |
| Comparative Example 8 | 20 | 55 | 25 |

### Test Example 2: Dissolution test

A dissolution test was performed on the capsule formulations of Examples 4 to 14 and Comparative Examples 4 to 8 under the following test conditions.

### <Dissolution test conditions>

1) Dissolution test method: Dissolution method II (paddle method) in the U.S. Pharmacopoeia (USP)
2) Dissolution medium: 500 mL of artificial gastric juice (pH 1.2)+ 1 mL of Tween 80 Solution
3) Amount of dissolution medium: 500 mL
4) Temperature of dissolution medium: 37 ± 0.5°C
5) Paddle speed: 100 rpm
6) No. of test samples: 6
7) Sampling and analysis method:
   About 10 mℓ of the dissolution medium was taken after 2 hours from the start of the dissolution test. After about 2.0 mℓ of an internal standard solution (a 0.0005% w/v solution of propylparaben in a mixture of water and acetonitrile (7:3)) was added to each of the sample solutions, the sample solutions were filtered using a membrane filter having a pore size of about 0.45 *µ*m or less. Each of the filtrates were analyzed by liquid chromatography (Column-Cosmosil^{®} C18 ODS (4.6 x 150 mm, 5 *µ*m), Temperature - about 40°C, Mobile phase - a 7:3 mixture of aqueous perchloric acid solution (adjusted to about pH 2.0 with sodium hydroxide) and acetonitrile) at a flow rate of about 1.3 mℓ/min (Injection volume - about 250 *µ*ℓ) using a UV detector at about 225 nm.
8) Reference range of dissolution rate: about 13% to about 34 % (USP basis)

As results of the dissolution test, an average of the dissolution rates of tamsulosin hydrochloride of the capsule formulations of each example and a deviation thereof were shown in Table 4.

**[Table 4] Dissolution test result**

| Example | Dissolution rate (%) | Deviation |
|---|---|---|
| Example 4 | 16.7 | 2.0 |
| Example 5 | 20.6 | 3.0 |
| Example 6 | 19.8 | 1.8 |
| Example 7 | 23.8 | 3.1 |
| Example 8 | 25.6 | 1.4 |
| Example 9 | 28.9 | 3.0 |
| Example 10 | 27.3 | 1.7 |
| Example 11 | 30.1 | 2.8 |
| Example 12 | 29.2 | 1.3 |
| Example 13 | 31.0 | 1.2 |
| Example 14 | 31.1 | 3.1 |
| Comparative Example 4 | 34.8 | 1.1 |
| Comparative Example 5 | 13.0 | 2.3 |
| Comparative Example 6 | 31.0 | 7.9 |
| Comparative Example 7 | 29.8 | 8.6 |
| Comparative Example 8 | 38.8 | 11.3 |

Based on the test results, the capsule formulations of Examples 4 to 14 were found to have a dissolution rate that falls within the reference level of about 13% to about 34% according to the U.S. Pharmacopoeia (USP), within a dissolution rate deviation of about 3.5% or less, and thus have good uniformity between unit dosage forms. Meanwhile, the capsule formulations of Comparative Examples 4 and 8 failed to comply with the reference level according to the USP. In particular, the capsule formulation of Comparative Example 5 had an average dissolution rate satisfying the lower limit of the reference level of the USP, but having individual dissolution rates which are out of the reference range. Unlike the capsule formulations of Examples 4 to 14, the capsule formulations of Comparative Examples 6, 7, and 8 had a dissolution rate deviation of about 7.0% or greater, indicating poor uniformity between unit dosage forms, including samples having a dissolution rate not complying with the reference level of the USP.

### Test Example 3: Content uniformity test

A content uniformity test was performed under the following test conditions to evaluate the content uniformity of tamsulosin hydrochloride among capsules prepared according to Examples 4 to 14 and Comparative Examples 6 to 8.

### < Content uniformity test conditions >

1) Test method: a USP's test method of the uniformity of dosage units of tamsulosin hydrochloride capsule.
2) No. of test samples: 10
3) Reference level: 15 or less (USP basis)

**[Table 5] Content uniformity test results (Reference level: 15 or less)**

| Example | Evaluation value |
|---|---|
| Example 4 | 4.2 |
| Example 5 | 5.0 |
| Example 6 | 3.0 |
| Example 7 | 5.3 |
| Example 8 | 3.1 |
| Example 9 | 4.9 |
| Example 10 | 2.2 |
| Example 11 | 2.9 |
| Example 12 | 1.5 |
| Example 13 | 1.2 |
| Example 14 | 6.0 |
| Comparative Example 6 | 16.8 |
| Comparative Example 7 | 18.3 |
| Comparative Example 8 | 19.6 |

As a result of the content uniformity test, the capsules of Examples 4 to 14 were found to have good content uniformity of 15 or less satisfying the reference level prescribed in the USP, indicating that the capsule formulations according to embodiments, including the tamsulosin hydrochloride-containing sustained-release pellets including about 50wt% to about 100 wt% of particles having a particle size of about 0.50 mm to about 0.85 mm and less than about 15wt% of particles having a particle size less than about 0.50 mm, have good content uniformity among capsules.

In contrast, the capsules of Comparative Examples 6 to 8 exhibited severe content inconsistency among capsules, disqualifying the reference level prescribed in the USP. This is attributed to that pellets having a particle size of less than about 0.50 mm and pellets having a particle size of about 0.50 mm or greater are not uniformly mixed in the preparation of the capsules formulation, leading to an evaluation value of about 16 or greater disqualifying the reference level according to the USP.

### Example 15: Preparation of composite formulation including tamsulosin hydrochloride-containing sustained-release pellets

Sustained-release pellets containing tamsulosin hydrochloride were prepared in the same manner as in Example 1, according to the processes and compositions represented in Table 6.

**[Table 6]**

| Process | Ingredients | (mg/capsule) |
|---|---|---|
| Mixing, wet-grinding, extrusion, spheronizing, and sieving | Tamsulosin hydrochloride (Tamsulosin HCl) | 0.4 |
| | Poly(vinyl acetate) aqueous dispersion 30% | 66.5 (20.0 on solid basis) |
| | Microcrystalline cellulose | 124.5 |
| | Hypromellose 2280 (100,000 SR) | 5.1 |
| | Purified water | (65.0) |
| Polymeric coating | Poly(vinyl acetate) aqueous dispersion 30% | 5.5 (1.7 on solid basis) |
| | Povidone (K-30) | 0.4 |
| | Propylene glycol | 0.4 |
| | Purified water | (16.00) |
| Enteric coating | Methacrylic acid·ethyl acrylate copolymer ((MAA-EA Copolymer (1:1)) dispersion 30% | 6.3 (1.9 on solid basis) |
| | Triacetin | 0.5 |
| | Purified water | (40.00) |
| Final mixing | Sucrose stearate | 0.2 |
| Total | | 155.1 |

Tadalafil tablets were prepared as follows, according to the processes and the composition represented in Table 7. First, the ingredients for wet granulation represented in Table 7 were mixed using a high-speed mixer, and a binder solution was added thereto and wet-ground together. This mixture was then dried using a fluid bed dryer and then sieved, thereby to prepare wet granulates. The wet granules were mixed with excipients (post-mixing), and then finally mixed with magnesium stearate. This final mixture was tableted using a rotary tablet press (GRC-18, available from Sejong Pharmatech Co., Ltd., Korea) with a circular punch having a diameter of 5.5 mm, thereby preparing tablets. These tadalafil tablets were coated with a coating solution of Opadry^{®} II Yellow dispersed in purified water.

**[Table 7]**

| Process | Ingredients | (mg/capsule) |
|---|---|---|
| Wet granulation | Tadalafil | 5.0 |
| | D-Mannitol | 14.0 |
| | Hydroxypropylcellulose | 3.0 |
| Binder solution | Hydroxypropylcellulose | 0.2 |
| | Sodium lauryl sulfate | 0.3 |
| | Purified water | (5.0) |
| Post-mixing | Sodium starch glycolate | 4.5 |
| | D-Mannitol | 35.8 |
| | Microcrystalline cellulose | 17.1 |
| Final mixing | Magnesium stearate | 1.2 |
| Coating | Opadry^{®} II Yellow | 2.5 |
| | Purified water | (12.5) |
| **Total** | | **83.6** |

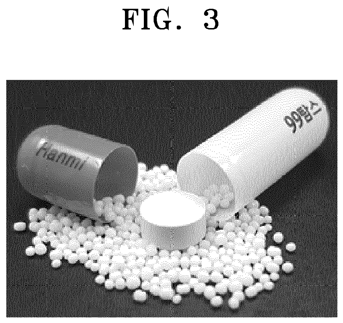

The tamsulosin hydrochloride-containing sustained-release pellets and the tadalafil tablets were filled into No. 1 hard capsules, thereby preparing composite capsule formulations each including 0.4 mg of tamsulosin and 5mg of tadalafil. The image of the contents including tamsulosin and tadalafil taken out of the prepared composite capsule formulation is shown in Fig. 3.

## Claims

1. A method of preparing an oral pharmaceutical formulation comprising sustained-release pellets containing tamsulosin hydrochloride and a pharmaceutically acceptable additive, the method comprising:
mixing and grinding a mixture of 10 parts to 300 parts by weight of polyvinyl acetate, 5 parts to 250 parts by weight of hydroxypropyl methylcellulose, and 1 part to 450 parts by weight of a diluting agent, with respect to 1 part by weight of tamsulosin hydrochloride, and 25wt% to 65wt% of distilled water based on a total weight of the forgoing ingredients, to form granules,
wherein the sustained-release pellets comprises 50 wt% to 100 wt% of particles having a particle size of 0.50 mm to 0.85 mm, and less than 15wt% of particles having a particle size less than 0.50 mm with respect to the total sustained-release pellets,
wherein the particle size is determined by sieving with a 0.85 mm sieve and a 0.5 mm sieve.

2. The method of claim 1, wherein the sustained-release pellets comprises 10 parts to 300 parts by weight of polyvinyl acetate, 5 parts to 250 parts by weight of hydroxypropyl methylcellulose, 1 part to 450 parts by weight of a diluting agent, each with respect to 1 part by weight of tamsulosin hydrochloride, and are prepared using 25 wt% to 65 wt% of distilled water with respect to a total weight of the forgoing ingredients.

3. The method of claim 1, wherein the sustained-release pellets comprises less than 50% of particles having a particle size of 0.85 mm or greater.

4. The method of claim 2, wherein the hydroxypropyl methylcellulose has a viscosity of 10,000 to 100,000 mPa·s (cPs).

5. The method of claim 2, wherein the diluting agent is selected from the group consisting of lactose, microcrystalline cellulose, dibasic calcium phosphate, dibasic calcium phosphate dihydrate, tribasic calcium phosphate, and any combinations thereof.

6. The method of claim 2, wherein the sustained-release pellets are additionally coated with a sustained-release coating agent.

7. The method of claim 6, wherein the sustained-release coating agent is a polymeric coating material or an enteric coating material.

8. The method of claim 1, wherein the oral pharmaceutical formulation is in the form of a capsule comprising the sustained-release granules.

9. The method of claim 1, wherein, as evaluated using Dissolution method II (paddle method) in the U.S. Pharmacopoeia (USP) in 500 mL of a pH1.2 aqueous buffer solution at 37±0.5°C at 100 rpm for 2 hours, the oral pharmaceutical formulation has a dissolution rate of the tamsulosin hydrochloride of 13% to 34% with a deviation of 4.0% or less

10. The method of claim 1, wherein an amount of the tamsulosin hydrochloride is 0.2 mg to 0.8 mg per single unit dosage form.

11. The method of claim 1, wherein the oral pharmaceutical formulation is for treatment of benign prostatic hypertrophy.

12. The method of claim 1, further comprising spheronizing the formed granules with a spheronizer at a rotation speed of 600 rpm to 800 rpm for 15 to 35 minutes to obtain spheronized pellets.

## Patentansprüche

1. Verfahren zum Herstellen einer oralen pharmazeutischen Formulierung, die Pellets mit verzögerter Freisetzung umfasst, welche Tamsulosinhydrochlorid und einen pharmazeutisch verträglichen Zusatzstoff enthalten, wobei das Verfahren umfasst:
Mischen und Mahlen einer Mischung aus 10 Gewichtsteilen bis 300 Gewichtsteilen Polyvinylacetat, 5 Gewichtsteilen bis 250 Gewichtsteilen Hydroxypropylmethylcellulose, und 1 Gewichtsteil bis 450 Gewichtsteilen eines Verdünnungsmittels, bezogen auf 1 Gewichtsteil Tamsulosinhydrochlorid, und 25 Gew.-% bis 65 Gew.-% destilliertes Wasser, bezogen auf ein Gesamtgewicht der vorstehenden Inhaltsstoffe, um Granulat zu bilden,
wobei die Pellets mit verzögerter Freisetzung 50 Gew.-% bis 100 Gew.-% Partikel mit einer Partikelgröße von 0,50 mm bis 0,85 mm, und weniger als 15 Gew.-% Partikel mit einer Partikelgröße von weniger als 0,50 mm, bezogen auf die Gesamtheit der Pellets mit verzögerter Freisetzung, umfassen,
wobei die Partikelgröße durch Sieben mit einem 0,85-mm-Sieb und einem 0,5-mm-Sieb bestimmt wird.

2. Verfahren nach Anspruch 1, wobei die Pellets mit verzögerter Freisetzung 10 Gewichtsteile bis 300 Gewichtsteile Polyvinylacetat, 5 Gewichtsteile bis 250 Gewichtsteile Hydroxypropylmethylcellulose, 1 Gewichtsteil bis 450 Gewichtsteile eines Verdünnungsmittels, jeweils bezogen auf 1 Gewichtsteil Tamsulosinhydrochlorid, umfassen, und unter Verwendung von 25 Gew.-% bis 65 Gew.-% destilliertem Wasser, bezogen auf ein Gesamtgewicht der vorstehenden Inhaltsstoffe, hergestellt werden.

3. Verfahren nach Anspruch 1, wobei die Pellets mit verzögerter Freisetzung weniger als 50% Partikel mit einer Partikelgröße von 0,85 mm oder größer umfassen.

4. Verfahren nach Anspruch 2, wobei die Hydroxypropylmethylcellulose eine Viskosität von 10.000 bis 100.000 mPas (cPs) aufweist.

5. Verfahren nach Anspruch 2, wobei das Verdünnungsmittel ausgewählt ist aus der Gruppe bestehend aus Laktose, mikrokristalliner Cellulose, dibasischem Calciumphosphat, dibasischem Calciumphosphatdihydrat, tribasischem Calciumphosphat, und Kombinationen davon.

6. Verfahren nach Anspruch 2, wobei die Pellets mit verzögerter Freisetzung zusätzlich mit einem Beschichtungsmittel für verzögerte Freisetzung beschichtet werden.

7. Verfahren nach Anspruch 6, wobei das Beschichtungsmittel für verzögerte Freisetzung ein polymeres Beschichtungsmaterial oder ein enterisches Beschichtungsmaterial ist.

8. Verfahren nach Anspruch 1, wobei die orale pharmazeutische Formulierung in Form einer Kapsel vorliegt, die das Granulat mit verzögerter Freisetzung umfasst.

9. Verfahren nach Anspruch 1, wobei die orale pharmazeutische Formulierung, unter Verwendung der Dissolution Method II (Blattrührer-Methode) in der US-Pharmakopöe (USP) in 500 ml einer wässrigen Pufferlösung mit einem pH-Wert von 1,2 bei 37 ± 0,5°C bei 100 U/min über 2 Stunden gemessen, eine Auflösungsrate des Tamsulosinhydrochlorids von 13% bis 34% mit einer Abweichung von 4,0% oder weniger aufweist.

10. Verfahren nach Anspruch 1, wobei eine Menge des Tamsulosinhydrochlorids pro monolithischer Arzneiform 0,2 mg bis 0,8 mg beträgt.

11. Verfahren nach Anspruch 1, wobei die orale pharmazeutische Formulierung zur Behandlung von benigner Prostatahypertrophie dient.

12. Verfahren nach Anspruch 1, das weiter das Sphäronisieren des gebildeten Granulats mit einem Sphäronisierer bei einer Drehzahl von 600 U/min bis 800 U/min über 15 bis 35 Minuten umfasst, um sphäronisierte Pellets zu erhalten.

## Revendications

1. Procédé de préparation d'une formulation pharmaceutique orale comportant des granules à libération prolongée contenant du chlorhydrate de tamsulosine et un additif pharmaceutiquement acceptable, le procédé comprenant :
le mélange et le broyage d'un mélange de 10 parties à 300 parties en poids de polyacétate de vinyle, de 5 parties à 250 parties en poids d'hydroxypropylméthylcellulose et de 1 partie à 450 parties en poids d'un agent diluant, par rapport à 1 partie en poids de chlorhydrate de tamsulosine, et de 25 % en poids à 65 % en poids d'eau distillée par rapport à un poids total des ingrédients précités, pour former des granulés,
dans lequel les granules à libération prolongée comportent 50% en poids à 100% en poids de particules présentant une taille de particule de 0,50 mm à 0,85 mm, et moins de 15% en poids de particules présentant une taille de particule inférieure à 0,50 mm par rapport au total des granules à libération prolongée,
dans lequel la taille de particule est déterminée par tamisage avec un tamis de 0,85 mm et un tamis de 0,5 mm.

2. Procédé selon la revendication 1, dans lequel les granules à libération prolongée comportent 10 parties à 300 parties en poids de polyacétate de vinyle, 5 parties à 250 parties en poids d'hydroxypropylméthylcellulose, 1 partie à 450 parties en poids d'un agent diluant, chacun par rapport à 1 partie en poids de chlorhydrate de tamsulosine, et sont préparés en utilisant 25% en poids à 65% en poids d'eau distillée par rapport à un poids total des ingrédients précités.

3. Procédé selon la revendication 1, dans lequel les granules à libération prolongée comportent moins de 50% de particules présentant une taille de particule supérieure ou égale à 0,85 mm.

4. Procédé selon la revendication 2, dans lequel l'hydroxypropylméthylcellulose présente une viscosité de 10 000 à 100 000 mPa·s (cPs).

5. Procédé selon la revendication 2, dans lequel l'agent diluant est choisi dans le groupe constitué par le lactose, la cellulose microcristalline, le phosphate de calcium dibasique, le phosphate de calcium dibasique dihydraté, le phosphate de calcium tribasique, et toute combinaison de ceux-ci.

6. Procédé selon la revendication 2, dans lequel les granules à libération prolongée sont en outre enrobés d'un agent d'enrobage à libération prolongée.

7. Procédé selon la revendication 6, dans lequel l'agent d'enrobage à libération prolongée est une substance d'enrobage polymère ou une substance d'enrobage entérique.

8. Procédé selon la revendication 1, dans lequel la formulation pharmaceutique orale se présente sous forme d'une capsule comportant les granulés à libération prolongée.

9. Procédé selon la revendication 1, dans lequel, tel qu'évalué à l'aide du procédé de dissolution II (procédé à palettes) de la pharmacopée américaine (USP) dans 500 ml d'une solution tampon aqueuse de pH 1,2 à 37±0,5°C à 100 tr/min pendant 2 heures, la formulation pharmaceutique orale présente un taux de dissolution du chlorhydrate de tamsulosine de 13% à 34% avec un écart inférieur ou égal à 4,0%.

10. Procédé selon la revendication 1, dans lequel une quantité du chlorhydrate de tamsulosine est de 0,2 mg à 0,8 mg par forme posologique unitaire.

11. Procédé selon la revendication 1, dans lequel la formulation pharmaceutique orale est destinée au traitement de l'hypertrophie bénigne de la prostate.

12. Procédé selon la revendication 1, comprenant en outre la sphéronisation des granulés formés avec un sphéronisateur à une vitesse de rotation de 600 tr/min à 800 tr/min pendant 15 à 35 minutes pour obtenir des granules sphéronisés.
